# EUROPEAN PATENT SPECIFICATION

(11) **EP 3 668 475 B1**
(45) Date of publication and mention of the grant of the patent: **02.12.2026**
(21) Application number: 18760214.9
(22) Date of filing: 13.08.2018
(51) Int. Cl.: A61J 1/20

(54) **SPINNING FEMALE LUER WITH THREADABLY REMOVABLE FEATURE**
DREHENDER LUER-INNENKEGEL MIT PER GEWINDE ABNEHMBAREM BESTANDTEIL
LUER FEMELLE TOURNANT DOTÉ D'UNE CARACTÉRISTIQUE AMOVIBLE PAR FILETAGE

(30) Priority: 15.08.2017 US 201762545597 P
(43) Date of publication of application: 24.06.2020
(73) Proprietor: Becton, Dickinson and Company, Franklin Lakes, NJ 07417 (US)
(72) Inventor: WEST, Robert, E., Basking Ridge New Jersey 07920 (US); CANCELLIERI, Jude, Oakland New Jersey 07436 (US); ERALP, Kivilcim, New York New York 10128 (US); KIM, Jayeon, River Edge New Jersey 07661 (US); MALBIN, Alicia, Ridgewood New Jersey 07450 (US); YEMANE-TEKESTE, Girum, Hackensack New Jersey 07601 (US)
(74) Representative: Ström & Gulliksson AB
(86) International application number: PCT/US2018/046470
(87) International publication number: WO 2019/036352

(56) References cited:
- WO-A1-2016/199133
- US-A1- 2015 297 453

## Description

### CROSS-REFERENCE TO RELATED APPLICATION

The present application claims priority to United States Provisional Application Serial No. 62/545,597, entitled "Spinning Female Luer with Threadably Removable Feature" filed August 15, 2017.

### BACKGROUND OF THE INVENTION

### Field of the Invention

The present invention, according to claim 1, relates to an adapter for a closed system transfer assembly that permits fluid delivery from a first fluid container to a second fluid container through the adapter. More specifically, the invention is directed to an adapter with a connection arrangement for engaging and disengaging the adapter from the fluid container.

### Description of Related Art

Healthcare workers, such as pharmacists and nurses, can be subject to acute and long term health risks upon repeated exposure to drugs or solvents which might escape into the air during drug preparation, drug administration, and/or other similar handling. This problem is particularly serious when cytotoxins, antiviral drugs, antibiotics, and radiopharmaceuticals are used by the healthcare workers. The health risks faced by exposure to these drugs can include the development of cancer, reproductive problems, genetic conditions, and other serious health concerns. Other hazardous areas may be sample taking, such as samples concerning virus infections or similar situations. When performing infusions, it is often necessary to inject a drug or other medical substance into the infusion fluid, inside an infusion bag, or other infusion fluid container. This is often done by means of penetrating a septum or other fluid barrier of an injection port on the infusion bag or on the infusion fluid line with a needle of a syringe filled with the medical fluid in question. However, even before this situation occurs, it may be necessary to transfer the medical fluid from a vial to a syringe and then from the syringe to a secondary container. In each of these steps, staff may be exposed to the medical fluid by means of contamination. Such contamination may be vaporized medical fluid or aerosol in the air. The contamination may contaminate the staff through their lungs or by vaporized medical fluid or aerosol in the air that condensates on the skin to penetrate the skin of the staff. Some medicaments are even known to penetrate protection gloves and, thereby, contaminate the staff.

Exposure to contaminations like this may, on a long term basis, give rise to alarmingly high concentrations of medicaments in the blood or the human body of the staff as described above. It has been understood that, due to the many transferring steps between e.g., vials, syringes, infusion systems, etc., the risk for contamination during the actual insertion and retraction of a needle from the container, e.g., a vial, needs to be contained. Closed system transfer devices (CSTD) have been developed to ensure that the medicament is contained in the transfer device during transfer of the medicament.

Generally, a CSTD includes an adapter (referred to hereinafter as a syringe adapter) for connection to a first fluid container, such as a syringe, and a second adapter (referred to hereinafter as a vial adapter) for connection to a vial, a second syringe, or a conduit providing fluid access to the patient's circulatory system. According to one arrangement, the healthcare practitioner may reconstitute a powdered or lyophilized compound with saline or some other reconstitution medium by attaching the syringe to the vial through the syringe adapter and the vial adapter. The practitioner reconstitutes the drug, aspirates the compound into the syringe, disconnects the adapter, and then attaches the syringe adapter and syringe attached thereto to a patient delivery device, such as an IV line, for administration to the patient.

One type of syringe adapter that can be used in a CSTD has a proximal end with a male or female luer-lock element that is arranged to be joined with a corresponding female or male luer-lock element of the syringe. The luer-lock element can be screwed into and unscrewed from the corresponding luer-lock element. It is desirable to prevent accidental or inadvertent unscrewing of the components, which could lead to the disconnection of the fluid passageway extending through the adapter. Such disconnection may result in a serious contamination risk for a patient and/or any other person in the vicinity of the disconnected CSTD. The issue of safety in administration of hazardous medical compounds is one that has been identified as being of critical importance by professional organizations and government agencies alike.

It is, therefore, desirable to provide a syringe adapter for enabling fluid transfer from the syringe to the syringe adapter, vial adapter, and/or second fluid container, such as a line connector/adaptor, by facilitating a positive connection of the connectors and avoiding inadvertent or accidental disconnection of the syringe and the fluid connector. Specifically, it is desirable that the syringe and the syringe adapter may be connected together via a simple intuitive connection activity. US20150297453 and WO2016199133 disclose prior art adapters.

### SUMMARY OF THE INVENTION

According to one aspect of the present disclosure, an adapter for connection with a fluid container includes an outer housing having a distal end, a proximal end, and a substantially cylindrical sidewall extending between the distal end and the proximal end, an inner member including a body rotatably inserted within the outer housing, a connector extending from the body configured to connect the adapter to a fluid container, and at least one grasping member extending from the inner member, the grasping member being configured for grasping by a user of the adapter, a first locking arrangement engageable with a distal end of the inner member and configured to restrict the inner member from rotating relative to the outer housing in a first direction, and a second locking arrangement engageable with a proximal surface of the inner member and configured to restrict the inner member from moving in a proximal direction relative to the outer housing. The adapter is transitionable between a disengaged state, in which the first locking arrangement and the second locking arrangement are not engaged with the inner member, a first fully engaged state in which the first locking arrangement engages the inner member, and a second fully engaged state in which the second locking arrangement engages the inner member.

According to another aspect of the present disclosure, the inner member is rotatable in both the first direction and the second direction when the connector is in the disengaged state. The inner member is transitionable from an extended position to a recessed position by applying a compressive force to the inner member. The first locking arrangement includes at least one tooth extending inward from an inner surface of the sidewall of the outer housing and a corresponding tooth on the body of the inner member configured to engage the tooth on the sidewall. The at least one tooth extending inward from the inner surface of the sidewall of the outer housing and the corresponding tooth on the body of the inner member include an angled portion and a vertical locking surface. The first locking arrangement includes a plurality of teeth extending around a circumferential inner surface of the sidewall of the outer housing and a plurality of corresponding teeth extending from a distal end of the body of the inner member. The second locking arrangement includes at least one inwardly extending locking tab connected to a portion of an inner surface of the sidewall of the outer housing and configured to engage the proximal surface of the inner member. The second locking arrangement includes at least two inwardly extending locking tabs positioned on opposing sides of the sidewall of the outer housing. The at least one locking tab includes a locking surface configured such that applying a compressive force to the inner member biases the tab outward to insert the inner member into the outer housing. The connector includes an outer surface with helical threads, configured to engage corresponding threads on an inner surface of a portion of the fluid container. The connector includes a luer connector configured to receive a corresponding luer connector of the fluid container. The at least one grasping member includes two curved flanges extending from a proximal surface of the inner member, each curved flange including a planar portion and an angled portion extending from each end of the planar portion. The curved flanges are configured for grasping by the user to prevent rotation of the inner member in a second direction, in which the second direction is opposite the first direction. The at least one grasping member includes two bumps extending from a proximal surface of the inner member, each bump having a substantially hemispherical shape. The bumps are configured for pressing by the user to prevent rotation of the inner member in a second direction, in which the second direction is opposite the first direction. The at least one grasping member includes two flanges positioned on the connector, each flange including a vertical portion that extends vertically along a side surface of the connector. The flanges are configured for grasping by the user to prevent rotation of the inner member in a second direction, in which the second direction is opposite the first direction. One of the flanges further includes a horizontal portion that extends horizontally from the connector and perpendicular to the vertical portion. The at least one grasping member includes a thumb stop extending horizontally from the connector and vertically from the body. The thumb stop is configured for grasping by the user to prevent rotation of the inner member in a second direction, in which the second direction is opposite the first direction. The at least one grasping member includes a groove defined in the body of the inner member. The groove is configured for grasping by the user to prevent rotation of the inner member in a second direction, in which the second direction is opposite the first direction.

In another aspect of the present disclosure, a method of disconnecting a fluid container to an adapter includes the steps of providing an adapter; grasping the at least one grasping member; moving the fluid container in an axial direction towards the adapter; and rotating the fluid container to disconnect the fluid container from the inner member of the adapter. The adapter including an outer housing having a distal end, a proximal end, and a substantially cylindrical sidewall extending between the distal end and the proximal end, an inner member including a body rotatably inserted within the outer housing, a connector extending from the body configured to connect the adapter to a fluid container, and at least one grasping member extending from the inner member, the grasping member being configured for grasping by a user of the adapter, a first locking arrangement engageable with a distal end of the inner member and configured to restrict the inner member from rotating relative to the outer housing in a first direction, and a second locking arrangement engageable with a proximal surface of the inner member and configured to restrict the inner member from moving in a proximal direction relative to the outer housing.

In another aspect of the present disclosure, the at least one grasping member includes two curved flanges extending from a proximal surface of the inner member, each curved flange including a planar portion and an angled portion extending from each end of the planar portion. The curved flanges are configured for grasping by the user to prevent rotation of the inner member in a second direction, in which the second direction is opposite the first direction. The at least one grasping member includes two flanges positioned on the connector, each flange including a vertical portion that extends vertically along a side surface of the connector. The flanges are configured for grasping by the user to prevent rotation of the inner member in a second direction, in which the second direction is opposite the first direction.

In another aspect of the present disclosure, an adapter for connection with a fluid container includes an outer housing having a distal end, a proximal end, and a sidewall extending between the distal end and the proximal end, a hub cover attached to the proximal end of the outer housing, an inner member including a rotatable body inserted within the outer housing and a connector extending from the body configured to connect the adapter to a fluid container, a first locking arrangement engageable with the outer housing and configured to restrict the inner member from rotating relative to the outer housing in a first direction, and a second locking arrangement engageable with the outer housing and configured to restrict the inner member from moving in a proximal direction relative to the outer housing. The adapter is transitionable between a disengaged state, in which both the first locking arrangement and the second locking arrangement are not engaged with the inner member, a first fully engaged state in which the first locking arrangement engages the outer housing, and a second fully engaged state in which the second locking arrangement engages the outer housing.

The hub cover is integrally formed on the proximal end of the outer housing and is configured to surround the rotatable body of the inner member when the inner member is connected to the outer housing. The hub cover is substantially flexible so as to contact the inner member to prevent rotation of the inner member relative to the outer housing. The inner member is rotatable in both the first direction and the second direction when the connector is in the disengaged state, wherein the inner member is prevented from rotating in a first direction when the connector is in the first fully engaged state; and wherein the inner member is prevented from retracting proximally out of the housing while permitting the inner member to rotate freely in the second fully engaged state. The inner member is transitionable from an extended position to a recessed position by applying a compressive force to the inner member. The first locking arrangement includes at least one tooth extending inward from an inner surface of the inner member and at least one recess defined in the proximal end of the outer housing configured to engage the tooth on the inner member. The at least one tooth extending inward from the inner member and the corresponding recess defined in the outer housing include an angled portion and a vertical locking surface. The second locking arrangement includes at least one inwardly extending locking tab connected to a portion of an inner surface of the inner member and configured to engage a locking protrusion extending from the proximal end of the outer housing. The at least one locking tab includes a locking surface configured such that applying a compressive force to the inner member biases the locking tab outward to insert the inner member into the outer housing. The sidewall of the outer housing defines at least one indentation to assist a user in gripping the adapter.

These and other features and characteristics of the present invention, as well as the methods of operation and functions of the related elements of structures and the combination of parts and economies of manufacture, will become more apparent upon consideration of the following description and the appended claims with reference to the accompanying drawings, all of which form a part of this specification, wherein like reference numerals designate corresponding parts in the various figures. It is to be expressly understood, however, that the drawings are for the purpose of illustration and description only and are not intended as a definition of the limits of the invention. As used in the specification and the claims, the singular form of "a", "an", and "the" include plural referents unless the context clearly dictates otherwise. The invention is defined by the appended claims.

### BRIEF DESCRIPTION OF THE DRAWINGS

**Fig. 1** is a perspective view of a closed system transfer device system according to an aspect of the invention;
**Fig 2****.** is a front perspective view of an adapter according to an aspect of the invention;
**Fig. 3** is a top perspective view of the adapter of **Fig. 2****;**
**Fig. 4** is a side view of the adapter of **Fig. 2****;**
**Fig. 5** is a top view of the adapter of **Fig. 2****;**
**Fig. 6** is a cross-sectional view of the adapter of **Fig. 2****;**
**Fig. 7** is a top perspective view of an outer housing of the adapter of **Fig. 2** according to an aspect of the invention;
**Fig. 8** is a cross-sectional view of the outer housing of **Fig. 7****;**
**Fig. 9** is a perspective view of an inner member of the adapter of **Fig. 2** according to an aspect of the invention;
**Fig. 10** is a side view of the inner member of **Fig. 9****;**
**Fig. 11** is a cross-sectional view of the inner member of **Fig. 9****;**
**Fig. 12** is a perspective view of an inner member according to another aspect of the invention;
**Fig. 13** is a side view of the inner member of **Fig. 12****;**
**Fig. 14** is a top view of the inner member of **Fig. 12****;**
**Fig. 15** is a perspective view of an inner member according to another aspect of the invention;
**Fig. 16** is a side view of the inner member of **Fig. 15****;**
**Fig. 17** is a top view of the inner member of **Fig. 15****;**
**Fig. 18** is a perspective view of an inner member according to another aspect of the invention;
**Fig. 19** is a side view of the inner member of **Fig. 18****;**
**Fig. 20** is a top view of the inner member of **Fig. 18****;**
**Fig. 21** is a side view of an alternative aspect of the inner member of **Fig. 18****;**
**Fig. 22** is a perspective view of an inner member according to another aspect of the invention;
**Fig. 23** is a perspective view of an inner member according to another aspect of the invention;
**Fig. 24** is a cross-sectional view of the inner member of **Fig. 23****,**
**Fig. 25** is a perspective view of an adapter according to another aspect of the disclosure;
**Fig. 26** is a perspective view of the adapter of **Fig. 25** with an inner member removed;
**Fig. 27** is a cross-sectional view of the adapter of **Fig. 25****;**
**Fig. 28** is an enlarged view of an outer housing of the adapter of **Fig. 25****;**
**Fig. 29** is a bottom perspective view of the inner member of the adapter of **Fig. 25****;**
**Fig. 30** is a side view of another aspect of the adapter according to the present disclosure;
**Fig. 31** is a perspective view of an inner member according to another aspect of the present disclosure; and
**Fig. 32** is a perspective view of a hub cover according to another aspect of the present disclosure.

### DESCRIPTION OF THE INVENTION

The illustrations generally show preferred and non-limiting aspects of the systems and methods of the present disclosure. While the descriptions present various aspects of the devices, it should not be interpreted in any way as limiting the disclosure. Furthermore, modifications, concepts, and applications of the disclosure's aspects are to be interpreted by those skilled in the art as being encompassed by, but not limited to, the illustrations and descriptions herein.

Further, for purposes of the description hereinafter, the terms "end", "upper", "lower", "right", "left", "vertical", "horizontal", "top", "bottom", "lateral", "longitudinal", and derivatives thereof shall relate to the disclosure as it is oriented in the drawing figures. However, it is to be understood that the disclosure may assume various alternative variations and step sequences, except where expressly specified to the contrary. It is also to be understood that the specific devices and processes illustrated in the attached drawings, and described in the following specification, are simply exemplary aspects of the disclosure. Hence, specific dimensions and other physical characteristics related to the aspects disclosed herein are not to be considered as limiting. For the purpose of facilitating understanding of the disclosure, the accompanying drawings and description illustrate preferred aspects thereof, from which the disclosure, various aspects of its structures, construction and method of operation, and many advantages may be understood and appreciated.

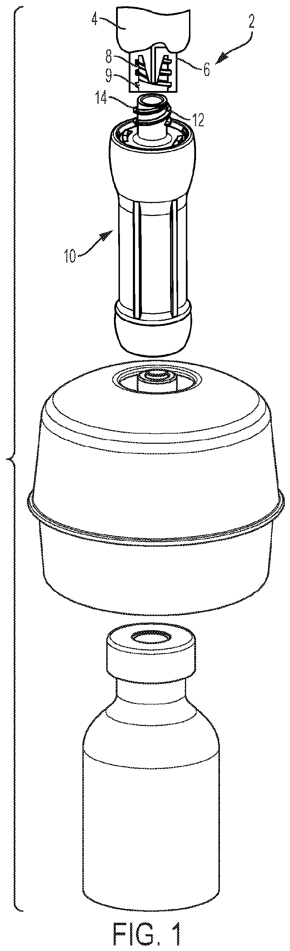

With reference to **Fig. 1****,** a closed system transfer assembly **2** is illustrated. The closed system transfer assembly **2** includes a first fluid source or container, such as a syringe **4** or IV line, configured to be connected to a syringe adapter (referred to hereinafter as adapter **10**). The syringe **4** includes a male luer connector **6** that is configured to be secured to a corresponding female luer-lock connector **12** of the adapter **10.** However, it is understood that the arrangement of the male and female luer-lock fittings may be reversed for certain fluid delivery applications. Any other connection interface, as is known in the art, may also be added in place of the luer fittings as required. The distal end of the syringe **4** may also include a luer-lock **8** surrounding the male luer connector **6** with threads **9** configured to engage corresponding threads **14** surrounding the connector **12.** More specifically, the adapter **10** is an assembly of components adapted to create a tamper-proof connection interface with the syringe **4.** The adapter **10** is configured to prevent accidental or inadvertent disconnection of the adapter **10** and the syringe **4,** which could compromise the integrity of the closed system transfer assembly **2.** As will be described in detail hereinafter, the adapter **10** includes various locking arrangements for preventing a user from inadvertently disengaging the adapter **10** from the syringe **4.** As a result of the locking arrangements, to disengage the syringe **4** from the adapter **10,** the user must perform a compound motion activity. As referred to hereinafter, a compound motion activity refers to more than one distinct and independent motion performed in a predetermined order or sequence. For example, in one aspect of the adapter **10,** the compound motion activity includes at least three distinct motions, namely pressing the syringe **4** toward the adapter **10,** pressing a button, tab, or surface located on the adapter **10,** and rotating the syringe **4** relative to the adapter **10** to disengage the threads **14** of the connector **10** from the threads **9** on the luer-lock **8** of the syringe **4.** The sequence of predetermined steps may also be reversed or performed in a different order within the scope of the present invention.

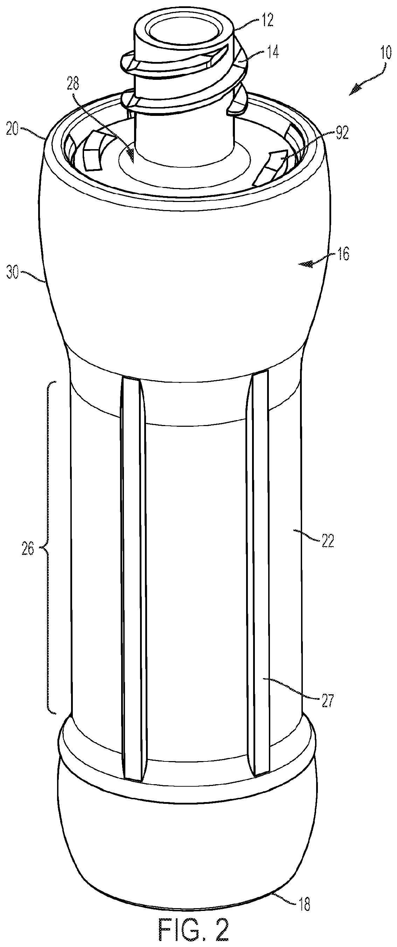

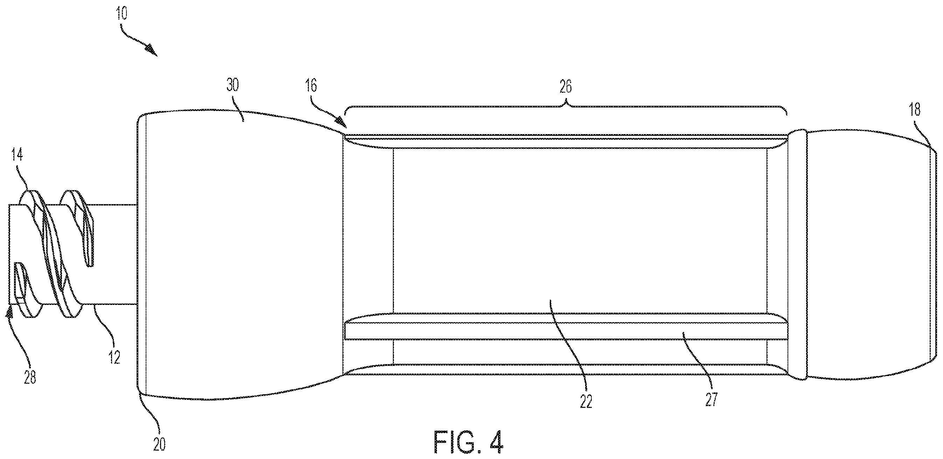

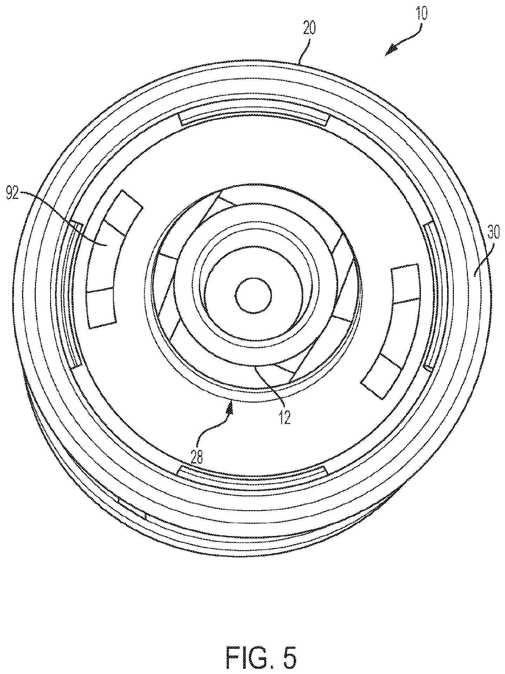

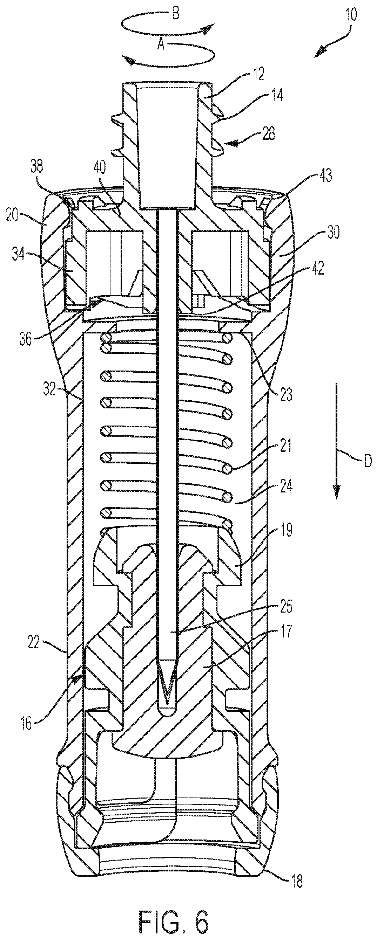

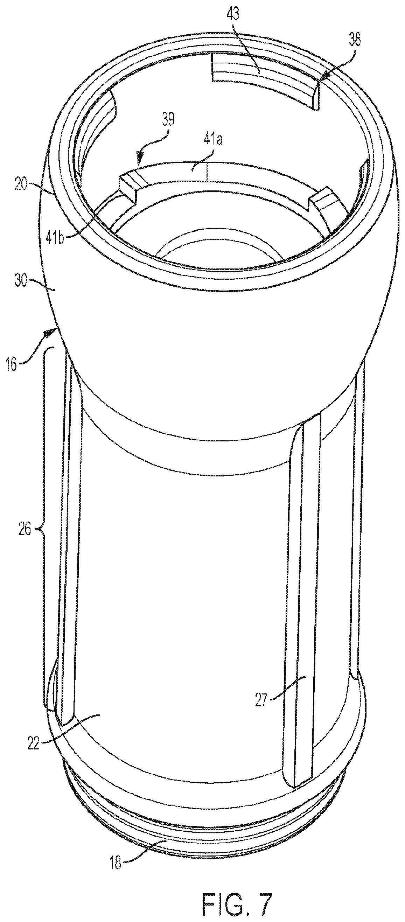

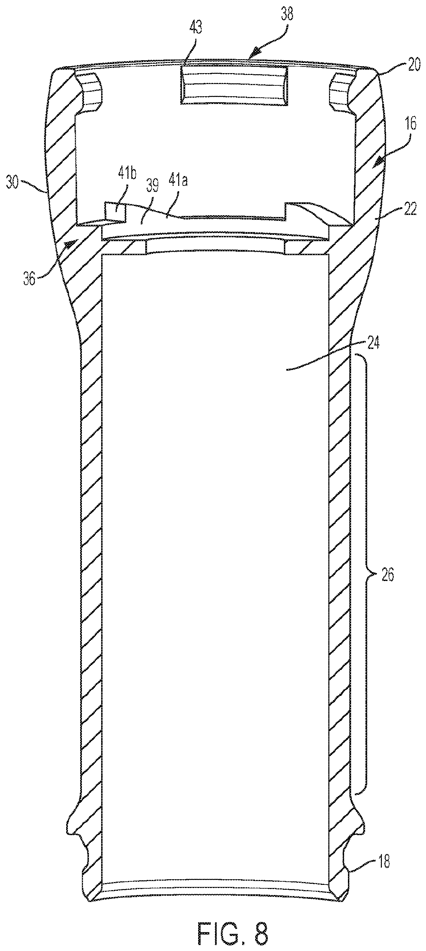

With reference to **Figs. 2-8****,** the adapter **10** includes an outer housing **16** having a distal end **18,** a proximal end **20,** and a generally cylindrical sidewall **22** extending between the distal end **18** and the proximal end **20.** The housing **16** defines a fluid passageway **24** (shown in **Fig. 6**) extending between the proximal end **20** and distal end **18** of the outer housing **16.** The housing **16** may be formed from any suitable structural material including medical grade plastic or metal. Optionally, the housing **16** may include various features that make holding or manipulating the housing **16** and adapter **10** easier. For example, the housing **16** may include a narrower grip portion **26** that is more comfortable for users to hold. The housing **16** may also include a plurality of ribs **27** or a textured portion or surface (not shown) so that the housing **16** does not slip or slide when held by the user. The housing **16** may also include various aesthetic features such as patterns, designs, logos, and the like for improving the appearance of the outer housing **16.**

With reference to **Fig. 6****,** in certain aspects, the housing **16** includes a needle cannula **25** extending therethrough that forms the fluid passageway **24.** The cannula **25** may include a tip at a distal end thereof for establishing a fluid connection with a fluid container such as a medical vial. The housing **16** may also include a septum **17** or seal arrangement, capable of being pierced by the tip of the needle, extending across an inner portion of the housing **16.** The septum **17** is held within the housing **16** by a holding member **19** that rests on the distal end **18** of the housing **16.** A resilient member, such as a spring **21,** is also positioned in the housing **16** and rests against a proximal end of the holding member **19** and a flange **23** extending from an inner surface of the housing **16.** The spring **21** is biased against the holding member **19** to keep the septum **17** positioned in the housing **16.** During use, the needle tip and cannula **25** may be advanced through the septum **17** or seal arrangement to establish fluid communication through the housing **16.** The septum **17** or seal arrangement may be configured to prevent fluid from passing through the housing **16** and contaminating other elements of the adapter **10** and/or syringe **4.**

The adapter **10** further includes an inner member **28** inserted in the proximal end **20** of the housing **16.** For example, in one aspect, the inner member **28** may be inserted in an annular sleeve **30** extending around the proximal end **20** of the housing **16.** In one aspect, the inner member **28** rests on a proximal surface of the flange **23.** As will be described hereinafter, an inner surface **32** (shown in **Fig. 6**) of the sidewall **22** may include various structures for engaging the inner member **28** to restrict rotation thereof. The inner member **28** includes a substantially cylindrical body **34** having an outer diameter that generally corresponds with the inner diameter of the sidewall **22** of the housing **16.**

With reference to **Fig. 6****,** the body **34** of the inner member **28** is a substantially cylindrical structure, although other suitable shapes may be utilized. The body **34** may include a cap **40** or top on a proximal end thereof. The cap **40** covers a portion of the proximal end of the body **34.** The connector **12** extends from the cap **40** of the body **34.** A proximal end of the cannula **25** may be inserted into a proximal end of the connector **12** for permitting fluid flow through the housing **16** of the adapter **10.** A plurality of teeth **35** extend distally from a distal end **42** of the inner member **28** and are spread around the circumferential surface of the distal end **42** of the inner member **28.** Each tooth **35** includes an angled portion **37a** and a locking surface **37b** that extend substantially perpendicular to and end of the angled portion **37a.** It is also contemplated that fewer or additional teeth **35** may be provided on the inner member **28.**

The connector **12** includes various structures for connecting the inner member **28** of the adapter **10** to the syringe **4** (shown in **Fig. 1**). As described above, in one aspect, the exterior sidewall of the connector **12** includes helical threads **14** extending therefrom. The threads **14** are configured to engage corresponding threads **9** on the syringe **4** (shown in **Fig. 1**). For example, the user may connect the syringe **4** to the connector **10** by twisting the syringe **4** in the first direction A.

The adapter **10** also includes a first locking arrangement **36** that is capable of engaging the housing **16** with the body **34** of the inner member **28** for restricting the inner member **28** from turning in a first direction, such as clockwise, and a second locking arrangement **38** that is capable of engaging the housing **16** with the body **34** of the inner member **28** for restricting the inner member **28** from being retracted in a proximal direction out of the housing **16.** In one aspect, the first locking arrangement **36** includes a plurality of teeth **39** extending around an inner circumferential surface of the housing **16.** Each tooth **39** includes an angled portion **41a** and a locking surface **41b** that extends substantially perpendicular to and end of the angled portion **41a.**

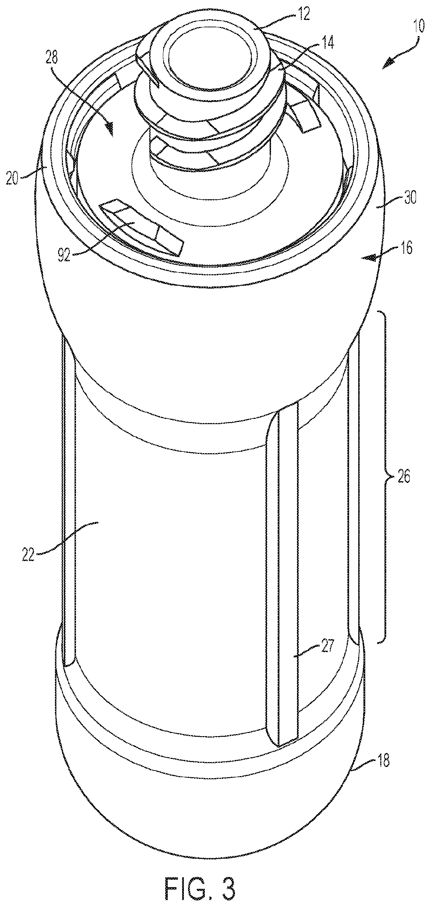

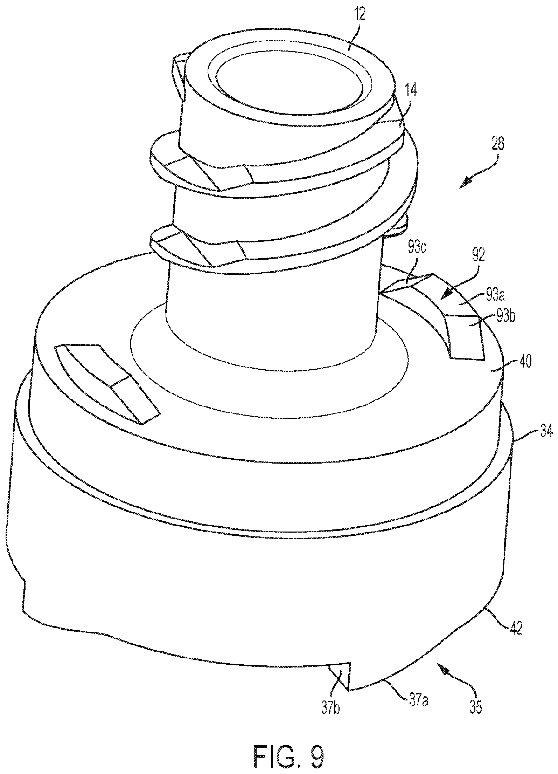

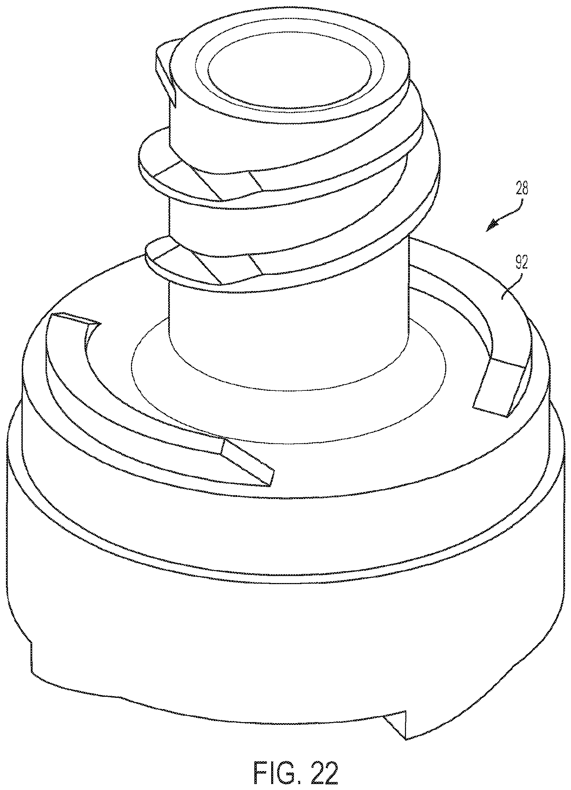

As shown in **Figs. 2****,** **3****,** and **9****,** grasping surfaces, such as one or more flanges **92,** may extend from the proximal surface of the cap **40** of the inner member **28.** As shown in **Fig. 9****,** in one aspect, two flanges **92** are provided on the cap **40** of the inner member **28.** The flanges **92** are positioned 180 degrees from one another on the cap **40.** In other aspects, fewer or additional flanges may be provided on the cap **40.** The flanges **92** may be positioned at any angular arrangement on the cap **40.** In one aspect, the flanges **92** are curved to extend around a portion of the circumferential surface of the cap **40.** As shown in **Fig. 22****,** the flanges **92** may extend along a larger portion of the cap **40.** When disconnecting the syringe **4** (as described below) from the inner member **28,** the user can grasp at least one flange **92** with one hand with sufficient force to prevent the inner member **28** from rotating. The grasping surfaces, such as the flanges **92** illustrated in **Fig. 3****,** may be easier for a user to hold for certain shapes of fluid sources or syringes. Each flange **92** includes a flat, planar portion **93a** and two angled portions **93b, 93c.** The angled portions **93b, 93c** extend from the proximal surface of the cap **40** to the planar portion **93a.** The user can grasp or press down on any portion of the flanges **92** to assist the user in preventing rotation of the inner member **28** relative to the housing **16** to disconnect the syringe **4** from the inner member **28.**

As will be described in greater detail hereinafter, the adapter **10** is transitionable between three states or positions. First, the adapter **10** may be in a disengaged state, in which the first locking arrangement **36** and the second locking arrangement **38** are not engaged with the inner member **28.** In the disengaged state, the inner member **28** can freely rotate relative to the stationary outer housing **16** in both the first direction A and the second direction B. Second, the adapter **10** may be in a first fully engaged state. In the first fully engaged state, the first locking arrangement **36** engages the inner member **28** so that rotation in the first direction A is substantially prevented. Finally, the adapter **10** may be transitioned to a second fully engaged state or position in which the second locking arrangement **38** engages the inner member **28,** thereby preventing the inner member **28** from retracting proximally out of the housing **16** while permitting the inner member **28** to rotate freely. It is noted, however, that some rotation may still occur in the partially engaged and fully engaged states if the locking arrangements **36, 38** have not reached a hard stop or if the user is not gripping the locking arrangements **36, 38** strongly enough to fully prevent rotation of the inner member **28.**

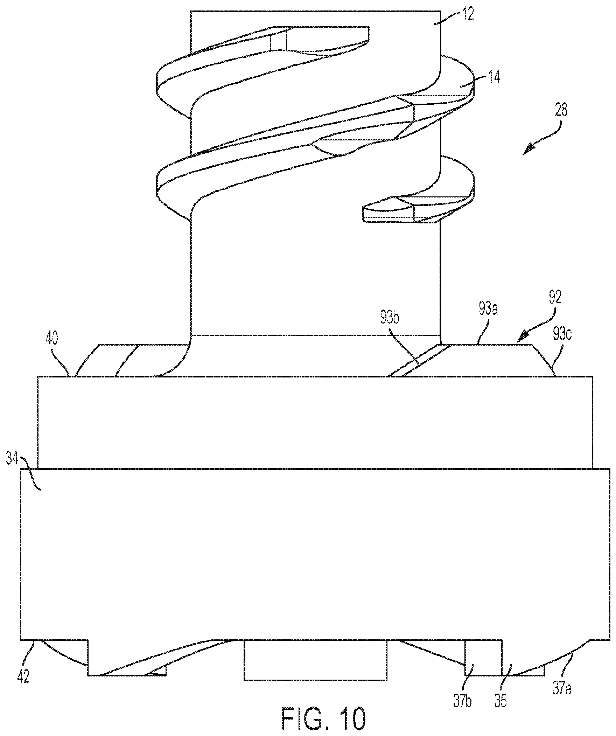

With reference to **Figs. 6** and **10****,** the inner member **28** is configured to be transitionable from an extended position to a recessed position in which the inner member **28** is inserted farther into the outer housing **16.** The user advances the inner member **28** in the distal direction D, relative to the housing **16,** to transition the inner member **28** from the extended position to the recessed position by applying a compressive force thereto. As will be described hereinafter, the first locking arrangement **36** cannot engage the inner member **28** when it is in the extended position. When the inner member **28** is in the recessed position, the first locking arrangement **36** is capable of engaging the inner member **28** to restrict rotation of the inner member **28** relative to the outer housing **16** in the first direction A. The inner member **28** is inserted into the housing **16** until the inner member **28** rests on the first locking arrangement **36.** After the inner member **28** has been positioned against the flange **23,** the inner member **28** is rotated or ratcheted until the teeth **35** extending from a distal end **42** of the inner member **28** engage the teeth **39** extending from the housing **16.** Upon interconnection between the teeth **35, 39,** the inner member **28** is permitted to rotate in the second direction B, but not the first direction A. The locking surfaces **37b** of the teeth **35** abut the locking surfaces **41b** of the first locking arrangement **36** to prevent rotation in the first direction A. During insertion and rotation of the inner member **28** within the housing **16,** the teeth **35** of the inner member **28** are directed upwards along the angled portions **41a** of the teeth **39** of the housing **16** so that the inner member **28** is permitted to freely rotate in the second direction B. However, when the inner member **28** is rotated in the first direction A, the locking surfaces **37b** of the teeth **35** of the inner member **28** abut the locking surfaces **41b** of the teeth **39** of the housing **16,** such that rotation of the inner member **28** in the first direction A is prevented. Therefore, as a practitioner rotates the syringe **4** onto the inner member **28,** the inner member **28** is prevented from rotating in the first or clockwise direction A to hold the inner member **28** stationary relative to the syringe **4** so the syringe **4** can be twisted onto the inner member **28.** However, since the inner member **28** is not prevent from rotating in the second direction B, the inner member **28** cannot be held stationary relative to the syringe **4** to permit the practitioner to rotate the syringe 4 in a counterclockwise direction to disconnect the syringe **4** from the inner member **28.** Using this arrangement, an unintentional or accidental disconnection of the syringe **4** from the inner member **28** is prevented.

Further, as the inner member **28** is inserted into the housing **16,** the second locking arrangement **38** engages the cap **40** of the inner member **28** to prevent proximal movement of the inner member **28** in the housing **16.** In one aspect, the second locking arrangement **38** includes a plurality of locking tabs **43** that extend around an inner circumferential surface of the housing **16.** As the inner member **28** is advanced into the housing **16,** the cap **40** is pushed past the locking tabs **43** to push the locking tabs **43** outwardly. Once the cap **40** advances past the locking tabs **43,** the locking tabs **43** move inwardly to engage the upper surface of the cap **40,** thereby preventing proximal movement of the inner member **28** out of the housing **16.**

As described above, the adapter **10** of the present disclosure is configured to require a compound motion or activity to disconnect the syringe **4** (shown in **Fig. 1**) from the adapter **10.** In a preferred and non-limiting aspect of the adapter **10,** the first activation motion or maneuver is considered to be pressing the inner member **28** in the distal direction D with a compressive force that is sufficient to push the inner member **28** past the locking tabs **43.**

With reference to **Fig. 6****,** to connect the syringe **4** to the adapter **10,** the user grasps the syringe **4** in a conventional manner. The user aligns the distal portion of the syringe **4** with the connector **12** of the adapter **10,** such that helical threads **14** of the connector **12** contact corresponding threads **9** on the shield surrounding the male luer lock **6** of the syringe **4.** It is noted, however, that since the adapter **10** is in the disengaged position, the inner member **28** spins freely in the second direction B. Therefore, if the user were to try to turn the syringe **4** in a counterclockwise direction relative to the connector **12,** the inner member **28** would also rotate preventing connection therebetween. Instead, the user must press the syringe **4** against the connector **12** in distal direction D with sufficient compressive force to insert the inner member **28** into the housing **16.** Once sufficient force is applied, the inner member **28** is transitioned to the recessed position.

In the recessed position, the teeth **35** (shown in **Figs. 9** and **10**) of the first locking arrangement **36** and the teeth **39** of the housing **16** are brought into contact with one another. More specifically, once the inner member **28** is in the recessed position, the user can slightly rotate the inner member **28** relative to the housing **16** to established contact and/or engagement between the teeth **35** of the inner member **28** and the teeth **39** extending from the housing **16.** Once the engagement between the inner member **28** and the housing **16** is established, the inner member **28** is prevented from rotating any farther in the first direction A. Thus, the user can rotate the syringe **4** in direction A relative to the connector **12** to engage the threads **9** of the syringe **4** with the corresponding helical threads **14** of the connector **12.** Since the inner member **28** is fixedly engaged with the first locking arrangement **36,** twisting the syringe **4** in direction A does not cause the inner member **28** to rotate.

Once the syringe **4** is sufficiently tightly connected to the connector **12** of the inner member **28,** the user can release the syringe **4.** In this position, the inner member **28** and syringe **4** attached thereto can freely rotate in the first direction A and/or second direction B relative to the housing **16.** Furthermore, since the inner member **28** and/or syringe **4** rotate freely in the second direction B, it would be rather difficult or impossible for the user to remove the syringe **4** from the connector **12** of the inner member **28** when it is in the extended position. Thus, the chance that the user or patient could inadvertently remove the syringe **4** from the adapter **10** is effectively reduced.

To remove the syringe **4** from the adapter **10,** the user first pushes the syringe **4** toward the adapter **10,** in the same manner described above, to transition the inner member **28** from the extended position to the recessed position. This action is referred to as the first motion or maneuver. Specifically, to disconnect the syringe **4** from the connector **12,** the user must rotate the syringe **4** in the second direction B. However, when the adapter **10** is in the first fully engaged position in which it cannot rotate in the first direction A, the inner member **28** is free to rotate in the second direction B, meaning that removing the syringe **4** from the connector **12** would be difficult or prevented. Therefore, the user must press the cap **40** of the inner member **28** downwardly to press the inner member **28** against the flange **23.** Pressing the cap **40** is referred to as the second motion or maneuver. By pressing the inner member **28** against the first locking arrangement **36,** the inner member **28** and, thereby, the syringe **4** are prevented from rotating in either direction due to friction established between the inner member **28** and the first locking arrangement **36.** Since, in this position, the inner member **28** is prevented from rotating in the second direction B, the user can easily twist the syringe **4** in the second direction B to unscrew it from the connector **12.** Unscrewing the syringe **4** from the connector **12** is referred to as the third motion of maneuver.

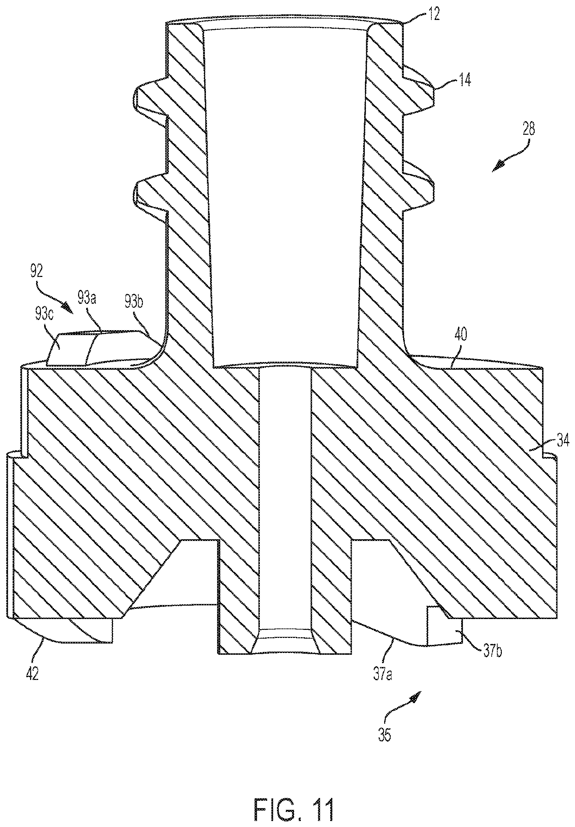

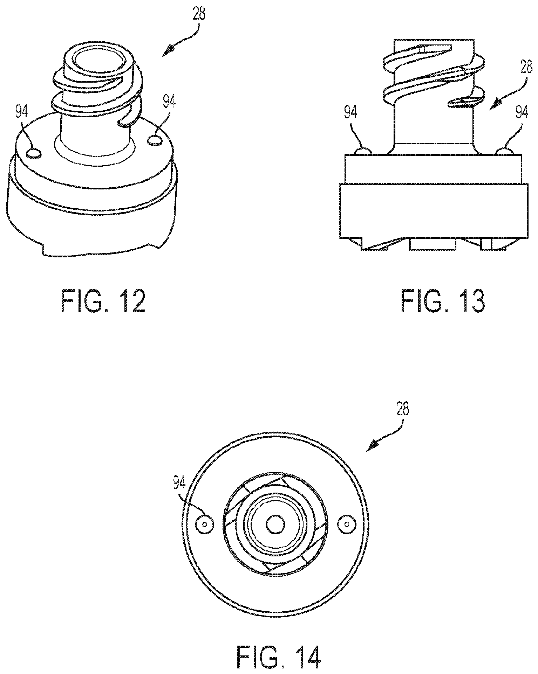

With reference to **Figs. 12-24****,** additional arrangements for preventing rotation of the inner member **28** relative to the housing **16** to disconnect the syringe **4** from the inner member **28** are shown and described. As shown in **Figs. 12-14****,** the inner member **28** includes the same features as the inner member **28** described above in **Figs. 1-11****.** The flanges **92** of the inner member **28,** however, are replaced with bumps **94** that extend from the proximal surface of the cap **40.** The bumps **94** have a hemispherical shape that is raised from the proximal surface of the cap **40.** The bumps **94** have substantially rounded edges to provide a smooth surface for a user to press down on when disconnecting the syringe **4** from the inner member **28.** In one aspect, two bumps **94** are provided on the cap **40.** The bumps **94** are spaced 180 degrees apart from one another on the cap **40.** It is also contemplated, however, that fewer or additional bumps may be provided on the cap **40** at any different angular arrangement.

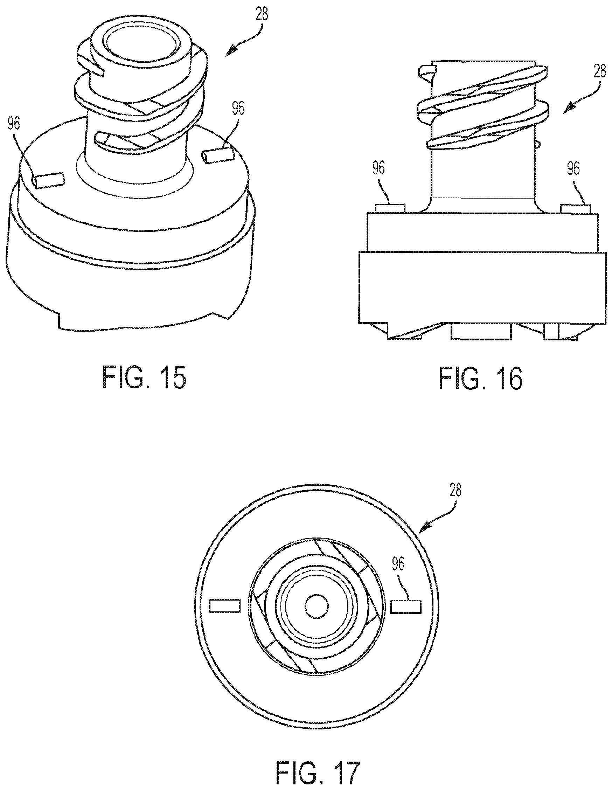

As shown in **Figs. 15-17****,** the inner member **28** includes the same features as the inner member **28** described above in **Figs. 1-11****.** The flanges **92** of the inner member **28,** however, are replaced with tabs **96** that extend from the proximal surface of the cap **40.** The tabs **96** include a planar portion that extends parallel to the proximal surface of the cap **40** and two curved portions that extend from the proximal surface of the cap **40** to connect to the planar portion. The curved portions have substantially rounded edges to provide a smooth surface for a user to press down on when disconnecting the syringe **4** from the inner member **28.** In one aspect, two tabs **96** are provided on the cap **40.** The tabs **96** are spaced 180 degrees apart from one another on the cap **40** and extend horizontally relative to the connector **12** of the inner member **28.** It is also contemplated, however, that fewer or additional tabs may be provided on the cap **40** at any different angular arrangement.

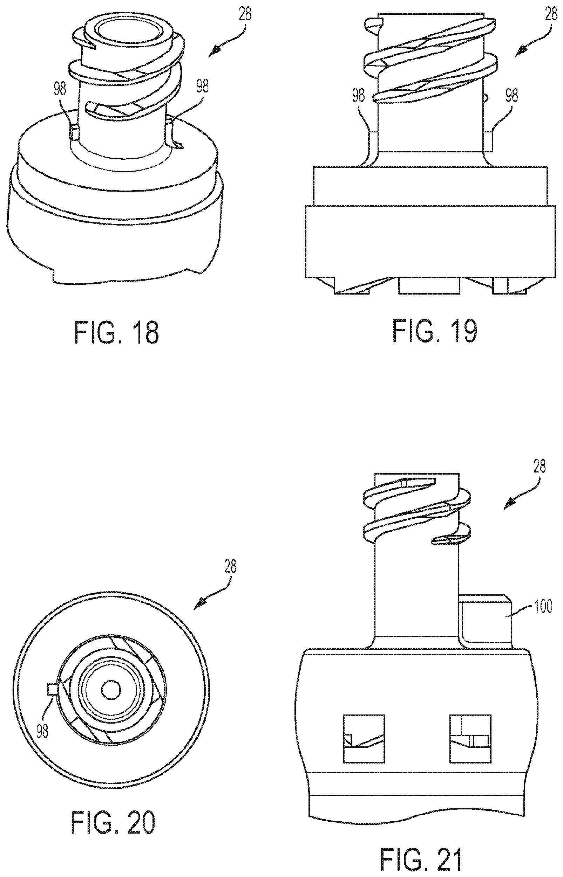

As shown in **Figs. 18-20****,** the inner member **28** includes the same features as the inner member **28** described above in **Figs. 1-11****.** The flanges **92** of the inner member **28,** however, are replaced with vertical flanges **98** that extend from the connector **12** of the inner member **28.** The vertical flanges **98** extend vertically along a portion of a side surface of the connector **12.** One of the vertical flanges **98** is substantially rectangular and includes a plurality of grasping surfaces that permit a user to stop rotation of the inner member **28** relative to the housing **16.** Another of the vertical flanges **98** includes a portion that extends vertically along a portion of the side surface of the connector **12** and a portion that extends horizontally along the proximal surface of the cap **40.** In this arrangement, the vertical flange **98** is positioned on the cap **40** and the connector **12.** In one aspect, two vertical flanges **98** are provided on the connector **12.** The vertical flanges **98** are spaced 180 degrees apart from one another on the connector **12** and extend vertically relative to the connector **12** of the inner member **28.** It is also contemplated, however, that fewer or additional vertical flanges may be provided on the connector **12** at any different angular arrangement. Further, as shown in **Fig. 21****,** a thumb stop **100** may be provided on one of the vertical flanges **98.** The thumb stop **100** may extend vertically from the cap **40** and horizontally from the connector **12** to provide a larger surface for the user to grasp to prevent rotation of the inner member **28** relative to the housing **16.** The user can press or hold the thumb stop **100** to prevent the inner member **28** from rotating in the second direction B to allow disconnection of the syringe **4** from the inner member **28.**

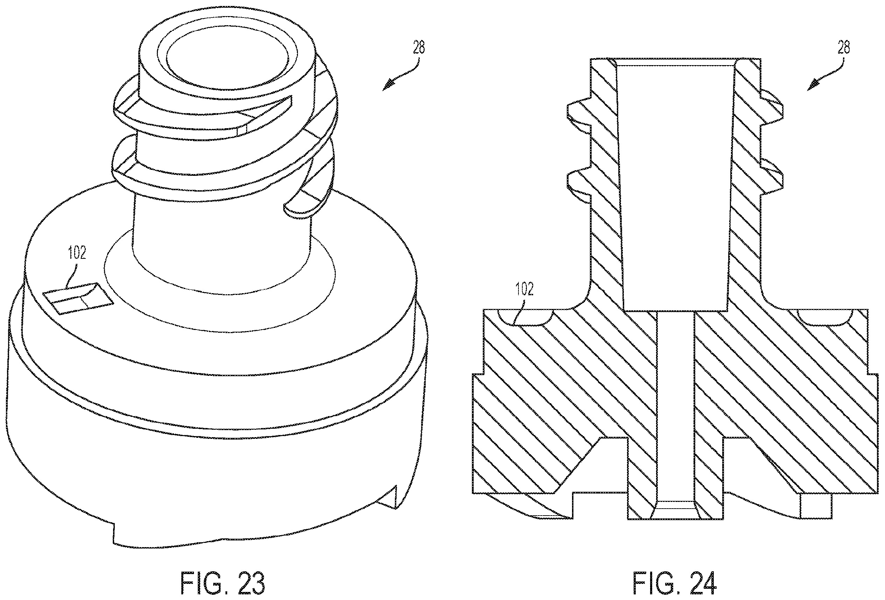

As shown in **Figs. 23** and **24****,** the inner member **28** includes the same features as the inner member **28** described above in **Figs. 1-11****.** The flanges **92** of the inner member **28,** however, are replaced with a groove **102** defined in the proximal surface of the cap **40.** The groove **102** includes a flat bottom surface, two curved side surfaces, and two vertical side surfaces. The groove **102** is configured to permit a user to insert a finger or tool into the groove **102** to prevent rotation of the inner member **28** relative to the housing **16** to disconnect the syringe **4** from the inner member **28.** By defining a groove **102** in the cap **40,** no external grasping surface extends from the proximal surface of the cap **40.** In one aspect, one groove **102** is defined in the cap **40.** It is also contemplated, however, that additional grooves may be defined in the cap **40** at any different angular arrangement.

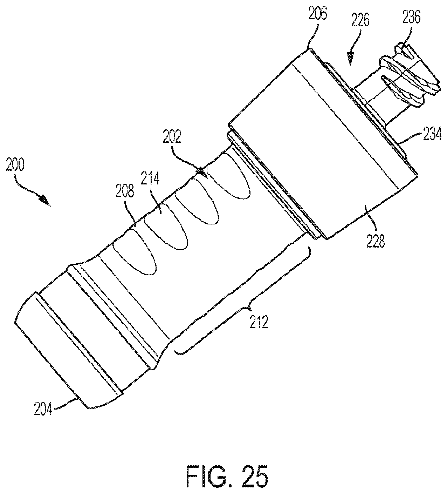

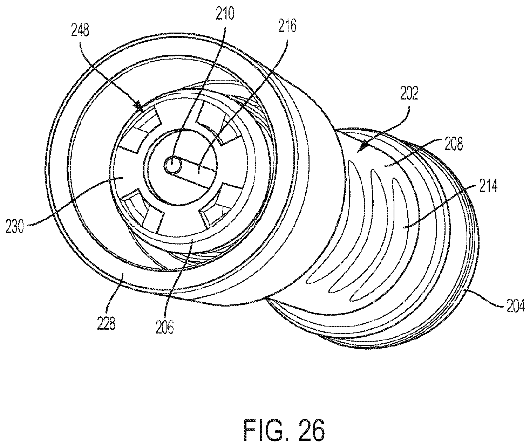

With reference to **Figs. 25-29****,** another aspect of the adapter **200** according to the present disclosure is described. The adapter **200** is similar in construction and function as the adapter **10** described above, but with a few differences as described below. The adapter **200** includes an outer housing **202** having a distal end **204,** a proximal end **206,** and a generally cylindrical sidewall **208** extending between the distal end **204** and the proximal end **206.** A needle extending through the housing **202** defines a fluid passageway **210** (shown in **Fig. 26**) extending between the proximal end **206** and distal end **204** of the outer housing **202.** The housing **202** may be formed from any suitable structural material including medical grade plastic or metal. Optionally, the housing **202** may include various features that make holding or manipulating the housing **202** and adapter **200** easier. For example, the housing **202** may include a narrower grip portion **212** that is more comfortable for users to hold. The housing **202** may also include a plurality of indentations **214** that are sized and shaped to receive a user's fingers during use of the adapter **200** so that the housing **202** does not slip or slide when held by the user. The housing **202** may also include various aesthetic features such as patterns, designs, logos, and the like for improving the appearance of the outer housing **202.**

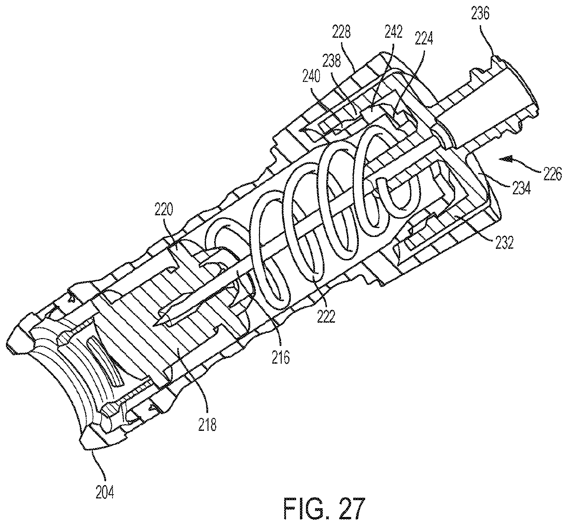

With reference to **Fig. 27****,** in certain aspects, the housing **202** includes a needle cannula **216** extending therethrough that forms the fluid passageway **210.** The cannula **216** may include a tip at a distal end thereof for establishing a fluid connection with a fluid container such as a medical vial. The housing **202** may also include a septum **218** or seal arrangement, capable of being pierced by the tip of the needle, extending across an inner portion of the housing **202.** The septum **218** is held within the housing **202** by a holding member **220** that rests on the distal end **204** of the housing **202.** A resilient member, such as a spring **222,** is also positioned in the housing **202** and rests against a proximal end of the holding member **220** and a flange **224** extending from an inner surface of the housing **202.** The spring **222** is biased against the holding member **220** to keep the septum **218** positioned in the housing **202.** During use, the needle tip and cannula **216** may be advanced through the septum **218** or seal arrangement to establish fluid communication through the housing **202.** The septum **218** or seal arrangement may be configured to prevent fluid from passing through the housing **202** and contaminating other elements of the adapter **200.**

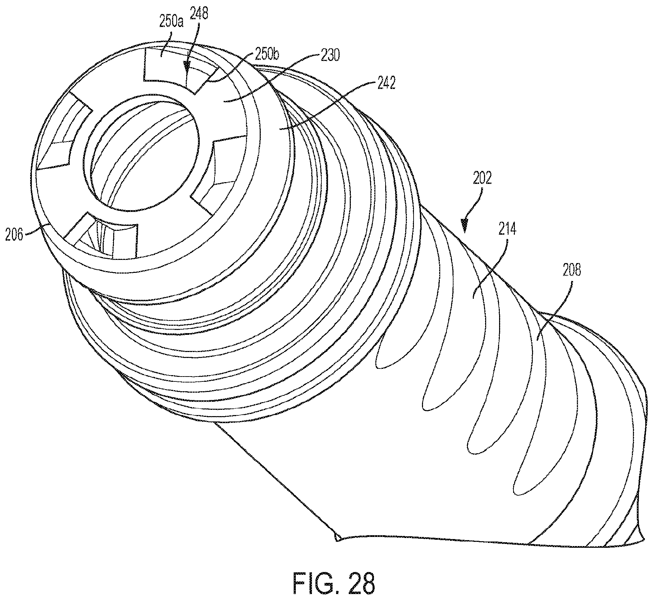

The adapter **200** further includes an inner member **226** inserted in the proximal end **206** of the housing **202.** For example, in one aspect, the inner member **226** may be inserted in a hub cover **228** extending around the proximal end **206** of the housing **202.** In one aspect, the inner member **226** rests on a proximal surface of the flange **224.** As will be described hereinafter, an inner surface **230** (shown in **Fig. 28**) of the sidewall **208** may include various structures for engaging the inner member **226** to restrict rotation thereof. The inner member **226** includes a substantially cylindrical body **232** having an outer diameter that generally corresponds with the inner diameter of the hub cover **228** of the housing **202.** The hub cover **228** is made of a substantially flexible material so that the hub cover **228** is configured to be pressed inwardly upon pressure applied by a user. After the pressure has been released by the user, the hub cover **228** will expand back to its original resting position. In one aspect, the hub cover **228** is made of a flexible, resilient plastic material.

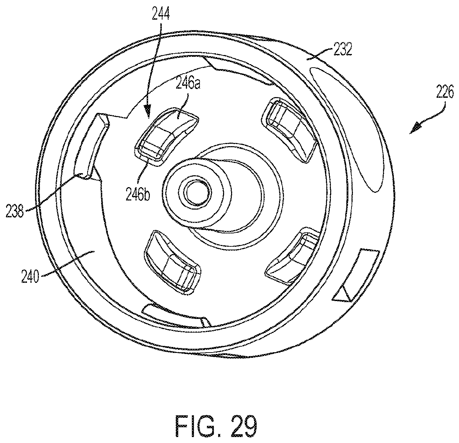

With reference to **Figs. 27** and **29****,** the body **232** of the inner member **226** is a substantially cylindrical structure, although other suitable shapes may be utilized. The body **232** may include a cap **234** or top on a proximal end thereof. The cap **234** covers a portion of the proximal end of the body **232.** A connector **236** extends from the cap **234** of the body **232** and is positioned such that the fluid passageway **210** extends therethrough. For example, a proximal end of the cannula **216** may be inserted into a proximal end of the connector **236** for permitting fluid flow through the needle of the adapter **200.** A plurality of locking tabs **238** (also referred to as a second locking arrangement) extend inwardly from an inner surface **240** of the inner member **236** and are spread around the circumferential surface of the inner surface **240** of the inner member **236.** As shown in **Fig. 27****,** when the adapter **200** is assembled, the inner member **226** is inserted into the hub cover **228.** As the inner member **226** is inserted into the hub cover **228,** the locking tabs **238** are pushed outwardly by sliding along a locking protrusion **242** that extends around an outer surface of the sidewall **208.** After the locking tabs **238** are pushed past the locking protrusion **242,** the locking tabs **238** snap back into place and are held a distal surface of the locking protrusion **242.** When in the locked position, the inner member **236** is prevented from being displaced proximally within the hub cover **228,** but is permitted to rotate within the hub cover **228.** Further, the inner surface **230** also prevents the spring **222** from biasing the inner member **226** upwards.

With reference to **Fig. 29****,** a first locking arrangement of the adapter **200** includes a plurality of teeth **244** extending from the proximal end of an inner surface of the inner member **226.** In one aspect, a total of four teeth **244** are provided on the inner surface of the inner member **226.** The teeth **244** are spread in a circular pattern around the inner surface of the inner member **226.** Each tooth **244** includes an angled portion **246a** and a locking surface **246b** that extend substantially perpendicular to an end of the angled portion **246a.** It is also contemplated that fewer or additional teeth **244** may be provided on the inner member **226.**

With reference to **Fig. 28****,** the proximal end **206** of the housing **202** includes a plurality of apertures **248** corresponding to the teeth **244** of the inner member **226.** The apertures **248** are defined in the proximal surface of the housing **202** and are configured to receive the teeth **244** of the inner member **226** when the inner member **226** is inserted in the hub cover **228.** The apertures **248** are spaced in a circular pattern in the proximal surface of the housing **202.** In one aspect, the apertures **248** extend through the entire proximal surface of the housing **202.** In another aspect, the apertures **248** only extend through a portion of the proximal surface of the housing **202.** In one aspect, four apertures **248** are defined in the proximal surface of the housing **202.** Each aperture **248** includes an angled portion **250a** and a locking surface **250b.** It is also contemplated that corresponding teeth may be used in place of the apertures **248.**

After the inner member **226** has been locked in the hub cover **228,** the teeth **244** of the inner member **226** and the apertures **248** defined in the housing **202** are configured to interact so as to permit rotation of the inner member **226** within the hub cover **228** in a counterclockwise direction and prevent rotation of the inner member **226** within the hub cover **228** in a clockwise direction. As the inner member **226** is rotated in the counterclockwise direction, the teeth **244** of the inner member **226** continue to slide along the angled portions **250a** of the apertures **248** of the housing **202** so that rotation of the inner member **226** is not prevented. Since the inner member **226** can continue to rotate in the counterclockwise direction, any accidental or inadvertent rotation of a fluid line connected to the connector **236** is also prevented. In one example, this accidental or inadvertent rotation of the fluid line may occur when a syringe or IV line is being attached. When the inner member **226** is rotated in the clockwise direction, the locking surface **246b** of the teeth **244** of the inner member **226** are held against the locking surfaces **250b** of the apertures **248** of the housing **202** so that rotation of the inner member **226** within the hub cover **228** is prevented. Since the inner member **226** is prevented from rotating in the clockwise direction, the inner member **226** remains stationary when a fluid line is being connected to the connector **236** of the inner member **226.**

When the user wishes to remove the fluid path line from the connector **236,** the user can grip and press the hub cover **228** inwardly against the inner member **226.** Since the hub cover **228** is pressed against the inner member **226,** the inner member **226** is prevented from rotating in either the clockwise or counterclockwise direction. Since the inner member **226** is held stationary in the counterclockwise direction in particular, the user can rotate the fluid line on the connector **236** in a counterclockwise direction to permit removal of the fluid line from the connector **236.** The removal of the fluid line from the connector **236** requires the user to actively grip and press the hub cover **228** against the inner member **226.** Using this configuration, the fluid line cannot inadvertently rotate on the connector **236** to allow for an inadvertent removal of the fluid line from the connector **236.**

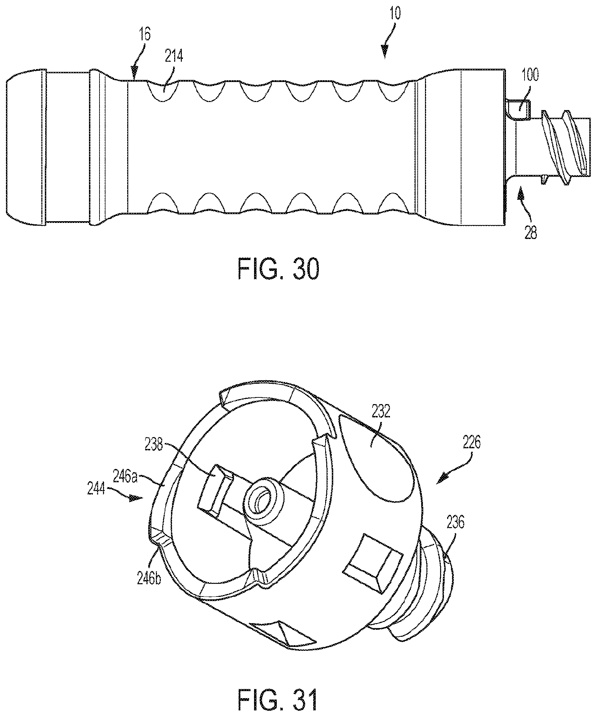

As shown in **Fig 30****,** in another aspect of the disclosure the grip indentations **214** provided on the housing **202** of the adapter **200** can also be provided on the housing **16** of the adapter **10** described above. Furthermore, in this aspect, the thumb stop **100** of **Fig. 21** can also be used on the inner member **28** of the adapter **10.**

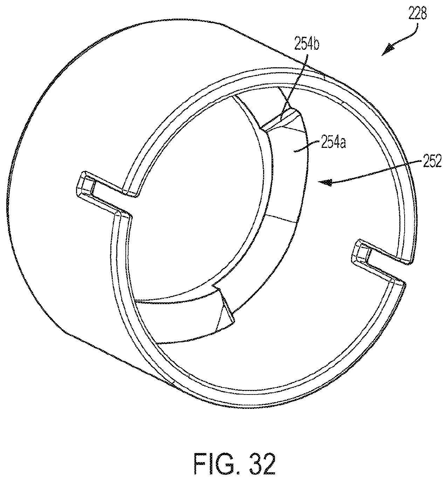

With reference to **Figs. 31** and **32****,** another locking arrangement between the inner member **226** and the hub cover **228** of the adapter **200** is described. As shown in **Fig. 31****,** instead of the teeth **244** being provided on a proximal inner surface of the inner member **226,** the teeth **244** are provided on a distal end of the inner member **226.** The teeth **244** are formed on the distal end of the inner member **226** and include the angled portion **246a** and the locking surface **246b.** As shown in **Fig. 32****,** corresponding teeth **252** are provided on an inner surface of the hub cover **228** to interact with the teeth **244** of the inner member **226** when the inner member **226** is locked in the hub cover **228.** The teeth **244** of the inner member **226** and the teeth **252** of the hub cover **228** interact to provide the same locking features that are provided by the teeth **244** of the inner member **226** and the apertures **248** of the housing **202.** The teeth **252** of the hub cover **228** include an angled portion **254a** and a locking surface **254b.** The locking surfaces **246b, 254b** of the inner member **226** and the hub cover **228,** respectively, are configured to interact to prevent clockwise rotation of the inner member **226** within the hub cover **228.**

Although the invention has been described in detail for the purpose of illustration based on what is currently considered to be the most practical and preferred aspects, it is to be understood that such detail is solely for that purpose and that the invention is not limited to the disclosed aspects, but, on the contrary, is intended to cover modifications that are within the scope of the appended claims. For example, it is to be understood that the present invention contemplates that, to the extent possible, one or more features of any aspect can be combined with one or more features of any other aspect.

## Claims

1. An adapter (10) for connection with a fluid container, the adapter (10) comprising:
an outer housing (16) having a distal end (18), a proximal end (20), and a sidewall (22) extending between the distal end (18) and the proximal end (20);
an inner member (28) comprising a rotatable body (34) inserted within the outer housing (16), a connector (12) extending from the body (34) configured to connect the adapter (10) to a fluid container (4), and at least one grasping member (92, 94, 96, 98, 100, 102) extending from the inner member (28);
a first locking arrangement (36) engageable with the inner member (28) and configured to restrict the inner member (28) from rotating relative to the outer housing (16) in a first direction (A), the first locking arrangement (36) comprises at least one tooth (39) extending inward from an inner surface (32) of the sidewall (22) of the outer housing (16) and a corresponding tooth (35) on the body (34) of the inner member (28) configured to engage the tooth (39) on the sidewall (22);
a second locking arrangement (38) engageable with the inner member (28) and configured to restrict the inner member (28) from moving in a proximal direction relative to the outer housing (16), the second locking arrangement (38) comprising at least one inwardly extending locking tab (43) extending from a portion of an inner surface (32) of the sidewall (22) of the outer housing (16) and configured to engage a proximal surface (40) of the inner member (28),
wherein the adapter (10) is transitionable between a disengaged state, in which both the first locking arrangement (36) and the second locking arrangement (38) are not engaged with the inner member (28), a first fully engaged state in which the first locking arrangement (36) engages the inner member (28), and a second fully engaged state in which the second locking arrangement (38) engages the inner member (28);
and wherein the inner member (28) is rotatable in both the first direction (A) and a second direction (B) opposite the first direction (A) when the connector (12) is in the disengaged state, wherein the inner member (28) is prevented from rotating in the first direction (A) when the connector (12) is in the first fully engaged state; and wherein the inner member (28) is prevented from retracting proximally out of the housing (16) while permitting the inner member (28) to rotate freely in the second fully engaged state.

2. The adapter (10) according to claim 1, wherein the inner member (28) is transitionable from an extended position to a recessed position by applying a compressive force to the inner member (28).

3. The adapter (10) according to claim 1, wherein the first locking arrangement (36) comprises a plurality of teeth (39) extending around a circumferential inner surface (32) of the sidewall (22) of the outer housing (16) and a plurality of corresponding teeth (35) extending from a distal end (42) of the body (34) of the inner member (28).

4. The adapter (10) according to claim 1, wherein the connector (12) comprises an outer surface with helical threads (14), configured to engage corresponding threads (9) on an inner surface of a portion of the fluid container (4).

5. The adapter (10) according to claim 1,
wherein the at least one grasping member (92, 94, 96, 98, 100, 102) comprises two curved flanges (92) extending from a proximal surface (40) of the inner member (28), each curved flange (92) including a planar portion (93a) and an angled portion (93b, 93c) extending from each end of the planar portion (93a), and
wherein the curved flanges (92) are configured for grasping by the user to prevent rotation of the inner member (28) in the second direction (B).

6. The adapter (10) according to claim 1,
wherein the at least one grasping member (92, 94, 96, 98, 100, 102) comprises two bumps (94) extending from a proximal surface (40) of the inner member (28), each bump (94) having a substantially hemispherical shape, and
wherein the bumps (94) are configured for pressing by the user to prevent rotation of the inner member (28) in the second direction (B).

7. The adapter (10) according to claim 1,
wherein the at least one grasping member (92, 94, 96, 98, 100, 102) comprises two flanges (98) positioned on the connector (12), each flange (98) comprising a vertical portion that extends vertically along a side surface of the connector (12), and
wherein the flanges (98) are configured for grasping by the user to prevent rotation of the inner member (28) in the second direction (B).

8. The adapter (10) according to claim 1,
wherein the at least one grasping member (92, 94, 96, 98, 100, 102) comprises a thumb stop (100) extending horizontally from the connector (12) and vertically from the body (34), and
wherein the thumb stop (100) is configured for grasping by the user to prevent rotation of the inner member (28) in the second direction (B).

9. The adapter (10) according to claim 1,
wherein the at least one grasping member (92, 94, 96, 98, 100, 102) comprises a groove (102) defined in the body (34) of the inner member (28), and
wherein the groove (102) is configured for grasping by the user to prevent rotation of the inner member (28) in the second direction (B).

10. A method of disconnecting a fluid container (4) to an adapter (10) comprising:
providing an adapter (10) comprising:
an outer housing (16) having a distal end (18), a proximal end (20), and a substantially cylindrical sidewall (22) extending between the distal end (18) and the proximal end (20);
an inner member (28) comprising a body (34) rotatably inserted within the outer housing (16), a connector (12) extending from the body (34) configured to connect the adapter (10) to a fluid container (4), and at least one grasping member (92, 94, 96, 98, 100, 102) extending from the inner member (28), the grasping member (92, 94, 96, 98, 100, 102) being configured for grasping by a user of the adapter (10);
a first locking arrangement (36) engageable with a distal end (42) of the inner member (28) and configured to restrict the inner member (28) from rotating relative to the outer housing (16) in a first direction (A), the first locking arrangement (36) comprises at least one tooth (39) extending inward from an inner surface (32) of the sidewall (22) of the outer housing (16) and a corresponding tooth (35) on the body (34) of the inner member (28) configured to engage the tooth (39) on the sidewall (22);
a second locking arrangement (38) engageable with a proximal surface (40) of the inner member (28) and configured to restrict the inner member (28) from moving in a proximal direction relative to the outer housing (16), the second locking arrangement (38) comprising at least one inwardly extending locking tab (43) extending from a portion of an inner surface (32) of the sidewall (22) of the outer housing (16) and configured to engage a proximal surface (40) of the inner member (28);
grasping the at least one grasping member (92, 94, 96, 98, 100, 102);
moving the fluid container (4) in an axial direction towards the adapter (10);
rotating the fluid container (4) to disconnect the fluid container (4) from the inner member (28) of the adapter (10);
wherein the adapter (10) is transitionable between a disengaged state, in which both the first locking arrangement (36) and the second locking arrangement (38) are not engaged with the inner member (28), a first fully engaged state in which the first locking arrangement (36) engages the inner member (28), and a second fully engaged state in which the second locking arrangement (38) engages the inner member (28);
and wherein
the inner member (28) is rotatable in both the first direction (A) and a second direction (B) opposite the first direction (A) when the connector (12) is in the disengaged state, wherein the inner member (28) is prevented from rotating in the first direction (A) when the connector (12) is in the first fully engaged state; and wherein the inner member (28) is prevented from retracting proximally out of the housing (16) while permitting the inner member (28) to rotate freely in the second fully engaged state.

11. The method according to claim 10,
wherein the at least one grasping member (92, 94, 96, 98, 100, 102) comprises two curved flanges (92) extending from a proximal surface (40) of the inner member (28), each curved flange (92) including a planar portion (93a) and an angled portion (93b, 93c) extending from each end of the planar portion (93a), and
wherein the curved flanges (92) are configured for grasping by the user to prevent rotation of the inner member (28) in the second direction (B).

12. The method according to claim 10,
wherein the at least one grasping member (92, 94, 96, 98, 100, 102) comprises two flanges (98) positioned on the connector (12), each flange (98) comprising a vertical portion that extends vertically along a side surface of the connector (12), and
wherein the flanges (98) are configured for grasping by the user to prevent rotation of the inner member (28) in the second direction (B).

## Patentansprüche

1. Ein Adapter (10) zur Verbindung mit einem Fluidbehälter, der Folgendes aufweist:
ein Außengehäuse (16), das ein distales Ende (18), ein proximales Ende (20) und eine Seitenwand (22) hat, die sich zwischen dem distalen Ende (18) und dem proximalen Ende (20) erstreckt;
ein Innenteil (28), das einen drehbaren Körper (34) aufweist, der in das Außengehäuse (16) eingesetzt ist, einen Verbinder (12), der sich von dem Körper (34) erstreckt und eingerichtet ist zum Verbinden des Adapters (10) mit einem Fluidbehälter (4), und wenigstens ein Greifelement (92, 94, 96, 98, 100, 102), das sich von dem Innenteil (28) erstreckt;
eine erste Verriegelungsanordnung (36), die mit dem Innenteil (28) in Eingriff bringbar und eingerichtet ist zum Beschränken der Drehung des Innenteils (28) relativ zu dem Außengehäuse (16) in einer ersten Richtung (A), wobei die erste Verriegelungsanordnung (36) wenigstens einen Zahn (39) aufweist, der sich von einer Innenfläche (32) der Seitenwand (22) des Außengehäuses (16) nach innen erstreckt, und einen entsprechenden Zahn (35) an dem Körper (34) des Innenteils (28), der eingerichtet ist zum Eingreifen in den Zahn (39) an der Seitenwand (22);
eine zweite Verriegelungsanordnung (38), die mit dem Innenteil (28) in Eingriff bringbar und eingerichtet ist zum Beschränken der Bewegung des Innenteils (28) in einer proximalen Richtung relativ zu dem Außengehäuse (16), wobei die zweite Verriegelungsanordnung (38) wenigstens eine sich nach innen erstreckende Verriegelungslasche (43) aufweist, die sich von einem Abschnitt einer Innenfläche (32) der Seitenwand (22) des Außengehäuses (16) erstreckt und eingerichtet ist zum Eingreifen in eine proximale Fläche (40) des Innenteils (28),
wobei der Adapter (10) zwischen einem gelösten Zustand, in dem sowohl die erste Verriegelungsanordnung (36) als auch die zweite Verriegelungsanordnung (38) nicht mit dem Innenteil (28) in Eingriff sind, einem ersten vollständig eingegriffenen Zustand, in dem die erste Verriegelungsanordnung (36) mit dem Innenteil (28) in Eingriff ist, und einem zweiten vollständig eingegriffenen Zustand, in dem die zweite Verriegelungsanordnung (38) mit dem Innenteil (28) in Eingriff ist, überführbar ist;
und wobei das Innenteil (28) sowohl in der ersten Richtung (A) als auch in einer zweiten Richtung (B), die der ersten Richtung (A) entgegengesetzt ist, drehbar ist, wenn der Verbinder (12) sich in dem gelösten Zustand befindet, wobei das Innenteil (28) daran gehindert wird, sich in der ersten Richtung (A) zu drehen, wenn der Verbinder (12) sich in dem ersten vollständig eingegriffenen Zustand befindet; und wobei das Innenteil (28) im zweiten vollständig eingegriffenen Zustand daran gehindert wird, sich proximal aus dem Gehäuse (16) zurückzuziehen, während es dem Innenteil (28) ermöglicht wird, sich frei zu drehen.

2. Der Adapter (10) nach Anspruch 1, wobei das Innenteil (28) durch Aufbringen einer Druckkraft auf das Innenteil (28) von einer ausgefahrenen Position in eine zurückgesetzte Position überführbar ist.

3. Der Adapter (10) nach Anspruch 1, wobei die erste Verriegelungsanordnung (36) eine Vielzahl von Zähnen (39) aufweist, die sich um eine umlaufende Innenfläche (32) der Seitenwand (22) des Außengehäuses (16) erstrecken, und eine Vielzahl von entsprechenden Zähnen (35), die sich von einem distalen Ende (42) des Körpers (34) des Innenteils (28) erstrecken.

4. Der Adapter (10) nach Anspruch 1, wobei der Verbinder (12) eine Außenfläche mit Spiralgewinde (14) aufweist, das eingerichtet ist zum Eingreifen in ein entsprechendes Gewinde (9) an einer Innenfläche eines Abschnitts des Fluidbehälters (4).

5. Der Adapter (10) nach Anspruch 1,
wobei das wenigstens eine Greifelement (92, 94, 96, 98, 100, 102) zwei gebogene Flansche (92) aufweist, die sich von einer proximalen Fläche (40) des Innenteils (28) erstrecken, wobei jeder gebogene Flansch (92) einen ebenen Abschnitt (93a) und einen abgewinkelten Abschnitt (93b, 93c) aufweist, der sich von jedem Ende des ebenen Abschnitts (93a) erstreckt, und
wobei die gebogenen Flansche (92) zum Ergreifen durch den Benutzer eingerichtet sind, um eine Drehung des Innenteils (28) in der zweiten Richtung (B) zu verhindern.

6. Der Adapter (10) nach Anspruch 1,
wobei das wenigstens eine Greifelement (92, 94, 96, 98, 100, 102) zwei Erhebungen (94) aufweist, die sich von einer proximalen Fläche (40) des Innenteils (28) erstrecken, wobei jede Erhebung (94) eine im Wesentlichen halbkugelförmige Form hat, und
wobei die Erhebungen (94) zum Drücken durch den Benutzer eingerichtet sind, um eine Drehung des Innenteils (28) in der zweiten Richtung (B) zu verhindern.

7. Der Adapter (10) nach Anspruch 1,
wobei das wenigstens eine Greifelement (92, 94, 96, 98, 100, 102) zwei Flansche (98) aufweist, die an dem Verbinder (12) angeordnet sind, wobei jeder Flansch (98) einen vertikalen Abschnitt aufweist, der sich vertikal entlang einer Seitenfläche des Verbinders (12) erstreckt, und
wobei die Flansche (98) zum Ergreifen durch den Benutzer eingerichtet sind, um eine Drehung des Innenteils (28) in der zweiten Richtung (B) zu verhindern.

8. Der Adapter (10) nach Anspruch 1,
wobei das wenigstens eine Greifelement (92, 94, 96, 98, 100, 102) einen Daumenanschlag (100) aufweist, der sich horizontal von dem Verbinder (12) und vertikal von dem Körper (34) erstreckt, und
wobei der Daumenanschlag (100) zum Ergreifen durch den Benutzer eingerichtet ist, um eine Drehung des Innenteils (28) in der zweiten Richtung (B) zu verhindern.

9. Der Adapter (10) nach Anspruch 1,
wobei das wenigstens eine Greifelement (92, 94, 96, 98, 100, 102) eine Nut (102) aufweist, die in dem Körper (34) des Innenteils (28) ausgebildet ist, und
wobei die Nut (102) zum Ergreifen durch den Benutzer eingerichtet ist, um eine Drehung des Innenteils (28) in der zweiten Richtung (B) zu verhindern.

10. Ein Verfahren zum Trennen eines Fluidbehälters (4) von einem Adapter (10), das Folgendes aufweist:
Bereitstellen eines Adapters (10), der Folgendes aufweist:
ein Außengehäuse (16), das ein distales Ende (18), ein proximales Ende (20) und eine im Wesentlichen zylindrische Seitenwand (22) hat, die sich zwischen dem distalen Ende (18) und dem proximalen Ende (20) erstreckt;
ein Innenteil (28), das einen Körper (34) aufweist, der drehbar in das Außengehäuse (16) eingesetzt ist, einen Verbinder (12), der sich von dem Körper (34) erstreckt und eingerichtet ist zum Verbinden des Adapters (10) mit einem Fluidbehälter (4), und wenigstens ein Greifelement (92, 94, 96, 98, 100, 102), das sich von dem Innenteil (28) erstreckt, wobei das Greifelement (92, 94, 96, 98, 100, 102) zum Ergreifen durch einen Benutzer des Adapters (10) eingerichtet ist;
eine erste Verriegelungsanordnung (36), die mit einem distalen Ende (42) des Innenteils (28) in Eingriff bringbar und eingerichtet ist zum Beschränken der Drehung des Innenteils (28) relativ zu dem Außengehäuse (16) in einer ersten Richtung (A), wobei die erste Verriegelungsanordnung (36) wenigstens einen Zahn (39) aufweist, der sich von einer Innenfläche (32) der Seitenwand (22) des Außengehäuses (16) nach innen erstreckt, und einen entsprechenden Zahn (35) an dem Körper (34) des Innenteils (28), der eingerichtet ist zum Eingreifen in den Zahn (39) an der Seitenwand (22);
eine zweite Verriegelungsanordnung (38), die mit einer proximalen Fläche (40) des Innenteils (28) in Eingriff bringbar und eingerichtet ist zum Beschränken der Bewegung des Innenteils (28) in einer proximalen Richtung relativ zu dem Außengehäuse (16), wobei die zweite Verriegelungsanordnung (38) wenigstens eine sich nach innen erstreckende Verriegelungslasche (43) aufweist, die sich von einem Abschnitt einer Innenfläche (32) der Seitenwand (22) des Außengehäuses (16) erstreckt und eingerichtet ist zum Eingreifen in eine proximale Fläche (40) des Innenteils (28);
Ergreifen des wenigstens einen Greifelements (92, 94, 96, 98, 100, 102);
Bewegen des Fluidbehälters (4) in einer axialen Richtung auf den Adapter (10) zu;
Drehen des Fluidbehälters (4) zum Trennen des Fluidbehälters (4) von dem Innenteil (28) des Adapters (10);
wobei der Adapter (10) zwischen einem gelösten Zustand, in dem sowohl die erste Verriegelungsanordnung (36) als auch die zweite Verriegelungsanordnung (38) nicht mit dem Innenteil (28) in Eingriff sind, einem ersten vollständig eingegriffenen Zustand, in dem die erste Verriegelungsanordnung (36) mit dem Innenteil (28) in Eingriff ist, und einem zweiten vollständig eingegriffenen Zustand, in dem die zweite Verriegelungsanordnung (38) mit dem Innenteil (28) in Eingriff ist, überführbar ist;
und wobei das Innenteil (28) sowohl in der ersten Richtung (A) als auch in einer zweiten Richtung (B), die der ersten Richtung (A) entgegengesetzt ist, drehbar ist, wenn der Verbinder (12) sich in dem gelösten Zustand befindet, wobei das Innenteil (28) daran gehindert wird, sich in der ersten Richtung (A) zu drehen, wenn der Verbinder (12) sich in dem ersten vollständig eingegriffenen Zustand befindet; und wobei das Innenteil (28) im zweiten vollständig eingegriffenen Zustand daran gehindert wird, sich proximal aus dem Gehäuse (16) zurückzuziehen, während es dem Innenteil (28) ermöglicht wird, sich frei zu drehen.

11. Das Verfahren nach Anspruch 10,
wobei das wenigstens eine Greifelement (92, 94, 96, 98, 100, 102) zwei gebogene Flansche (92) aufweist, die sich von einer proximalen Fläche (40) des Innenteils (28) erstrecken, wobei jeder gebogene Flansch (92) einen ebenen Abschnitt (93a) und einen abgewinkelten Abschnitt (93b, 93c) aufweist, der sich von jedem Ende des ebenen Abschnitts (93a) erstreckt, und
wobei die gebogenen Flansche (92) zum Ergreifen durch den Benutzer eingerichtet sind, um eine Drehung des Innenteils (28) in der zweiten Richtung (B) zu verhindern.

12. Das Verfahren nach Anspruch 10,
wobei das wenigstens eine Greifelement (92, 94, 96, 98, 100, 102) zwei Flansche (98) aufweist, die an dem Verbinder (12) angeordnet sind, wobei jeder Flansch (98) einen vertikalen Abschnitt aufweist, der sich vertikal entlang einer Seitenfläche des Verbinders (12) erstreckt, und
wobei die Flansche (98) zum Ergreifen durch den Benutzer eingerichtet sind, um eine Drehung des Innenteils (28) in der zweiten Richtung (B) zu verhindern.

## Revendications

1. Adaptateur (10) destiné à être connecté à un récipient de fluide, l'adaptateur (10) comprenant :
un boîtier externe (16) présentant une extrémité distale (18), une extrémité proximale (20) et une paroi latérale (22) s'étendant entre l'extrémité distale (18) et l'extrémité proximale (20) ;
un organe interne (28) comprenant un corps rotatif (34) inséré dans le boîtier externe (16), un connecteur (12) s'étendant à partir du corps (34) configuré pour connecter l'adaptateur (10) à un récipient de fluide (4), et au moins un organe de préhension (92, 94, 96, 98, 100, 102) s'étendant à partir de l'organe interne (28) ;
un premier agencement de verrouillage (36) pouvant venir en prise avec l'organe interne (28) et configuré pour restreindre la rotation de l'organe interne (28) par rapport au boîtier externe (16) dans une première direction (A), le premier agencement de verrouillage (36) comprenant au moins une dent (39) s'étendant vers l'intérieur à partir d'une surface interne (32) de la paroi latérale (22) du boîtier externe (16) et une dent (35) correspondante sur le corps (34) de l'organe interne (28) configurée pour venir en prise avec la dent (39) sur la paroi latérale (22) ;
un deuxième agencement de verrouillage (38) pouvant venir en prise avec l'organe interne (28) et configuré pour restreindre le déplacement de l'organe interne (28) dans une direction proximale par rapport au logement externe (16), le deuxième agencement de verrouillage (38) comprenant au moins une patte de verrouillage s'étendant vers l'intérieur (43) qui s'étend à partir d'une partie d'une surface interne (32) de la paroi latérale (22) du boîtier externe (16) et est configurée pour venir en prise avec une surface proximale (40) de l'organe interne (28), dans lequel l'adaptateur (10) peut permuter entre un état désolidarisé, dans lequel le premier agencement de verrouillage (36) et le deuxième agencement de verrouillage (38) ne sont pas en prise avec l'organe interne (28), un premier état entièrement en prise dans lequel le premier agencement de verrouillage (36) vient en prise avec l'organe interne (28), et un deuxième état entièrement en prise dans lequel le deuxième agencement verrouillage (38) vient en prise avec l'organe interne (28) ;
et dans lequel
l'organe interne (28) peut tourner à la fois dans la première direction (A) et dans une deuxième direction (B) opposée à la première direction (A) lorsque le connecteur (12) est dans l'état désolidarisé, dans lequel l'organe interne (28) est empêché de tourner dans la première direction (A) lorsque le connecteur (12) est dans le premier état entièrement en prise ; et dans lequel l'organe interne (28) est empêché de se rétracter proximalement hors du boîtier (16) tout en permettant à l'organe interne (28) de tourner librement dans le deuxième état entièrement en prise.

2. Adaptateur (10) selon la revendication 1, dans lequel l'organe interne (28) peut passer d'une position étendue à une position en retrait par application d'une force de compression à l'organe interne (28).

3. Adaptateur (10) selon la revendication 1, dans lequel le premier agencement de verrouillage (36) comprend une pluralité de dents (39) s'étendant autour d'une surface interne circonférentielle (32) de la paroi latérale (22) du boîtier externe (16) et une pluralité de dents (35) correspondantes s'étendant à partir d'une extrémité distale (42) du corps (34) de l'organe interne (28).

4. Adaptateur (10) selon la revendication 1, dans lequel le connecteur (12) comprend une surface externe avec des filetages hélicoïdaux (14), configurée pour venir en prise avec des filetages (9) correspondants sur une surface interne d'une partie du récipient de fluide (4).

5. Adaptateur (10) selon la revendication 1,
dans lequel l'au moins un organe de préhension (92, 94, 96, 98, 100, 102) comprend deux brides incurvées (92) s'étendant à partir d'une surface proximale (40) de l'organe interne (28), chaque bride incurvée (92) comportant une partie plane (93a) et une partie inclinée (93b, 93c) s'étendant à partir de chaque extrémité de la partie plane (93a), et
dans lequel les brides incurvées (92) sont configurées pour être saisies par l'utilisateur pour empêcher la rotation de l'organe interne (28) dans la deuxième direction (B).

6. Adaptateur (10) selon la revendication 1,
dans lequel l'au moins un organe de préhension (92, 94, 96, 98, 100, 102) comprend deux bosses (94) s'étendant à partir d'une surface proximale (40) de l'organe interne (28), chaque bosse (94) ayant une forme sensiblement hémisphérique, et
dans lequel les bosses (94) sont configurées pour être pressées par l'utilisateur pour empêcher la rotation de l'organe interne (28) dans la deuxième direction (B).

7. Adaptateur (10) selon la revendication 1,
dans lequel l'au moins un organe de préhension (92, 94, 96, 98, 100, 102) comprend deux brides (98) positionnées sur le connecteur (12), chaque bride (98) comprenant une partie verticale qui s'étend verticalement le long d'une surface latérale du connecteur (12), et
dans lequel les brides (98) sont configurées pour être saisies par l'utilisateur pour empêcher la rotation de l'organe interne (28) dans la deuxième direction (B).

8. Adaptateur (10) selon la revendication 1,
dans lequel l'au moins un organe de préhension (92, 94, 96, 98, 100, 102) comprend une butée de pouce (100) s'étendant horizontalement à partir du connecteur (12) et verticalement à partir du corps (34), et
dans lequel la butée de pouce (100) est configurée pour être saisie par l'utilisateur pour empêcher la rotation de l'organe interne (28) dans la deuxième direction (B).

9. Adaptateur (10) selon la revendication 1,
dans lequel l'au moins un organe de préhension (92, 94, 96, 98, 100, 102) comprend une rainure (102) définie dans le corps (34) de l'organe interne (28), et
dans lequel la rainure (102) est configurée pour être saisie par l'utilisateur pour empêcher la rotation de l'organe interne (28) dans la deuxième direction (B).

10. Procédé de déconnexion d'un récipient de fluide (4) d'un adaptateur (10) comprenant :
la fourniture d'un adaptateur (10) comprenant :
un boîtier externe (16) présentant une extrémité distale (18), une extrémité proximale (20) et une paroi latérale sensiblement cylindrique (22) s'étendant entre l'extrémité distale (18) et l'extrémité proximale (20) ;
un organe interne (28) comprenant un corps (34) inséré en rotation dans le boîtier externe (16), un connecteur (12) s'étendant à partir du corps (34) étant configuré pour connecter l'adaptateur (10) à un récipient de fluide (4), et au moins un organe de préhension (92, 94, 96, 98, 100, 102) s'étendant à partir de l'organe interne (28), l'élément de préhension (92, 94, 96, 98, 100, 102) étant configuré pour être saisi par un utilisateur de l'adaptateur (10) ;
un premier agencement de verrouillage (36) pouvant venir en prise avec une extrémité distale (42) de l'organe interne (28) et configuré pour restreindre la rotation de l'organe interne (28) par rapport au boîtier externe (16) dans une première direction (A), le premier agencement de verrouillage (36) comprenant au moins une dent (39) s'étendant vers l'intérieur à partir d'une surface interne (32) de la paroi latérale (22) du boîtier externe (16) et une dent (35) correspondante sur le corps (34) de l'organe interne (28) configurée pour venir en prise avec la dent (39) sur la paroi latérale (22) ;
un deuxième agencement de verrouillage (38) pouvant venir en prise avec une surface proximale (40) de l'organe interne (28) et configuré pour restreindre le déplacement de l'organe interne (28) dans une direction proximale par rapport au logement externe (16), le deuxième agencement de verrouillage (38) comprenant au moins une patte de verrouillage s'étendant vers l'intérieur (43) qui s'étend à partir d'une partie d'une surface interne (32) de la paroi latérale (22) du boîtier externe (16) et est configurée pour venir en prise avec une surface proximale (40) de l'organe interne (28) ;
la préhension de l'au moins un organe de préhension (92, 94, 96, 98, 100, 102) ;
le déplacement du récipient de fluide (4) dans une direction axiale vers l'adaptateur (10) ;
la rotation du récipient de fluide (4) pour déconnecter le récipient de fluide (4) de l'organe interne (28) de l'adaptateur (10) ;
dans lequel l'adaptateur (10) peut permuter entre un état désolidarisé, dans lequel le premier agencement de verrouillage (36) et le deuxième agencement de verrouillage (38) ne sont pas en prise avec l'organe interne (28), un premier état entièrement en prise dans lequel le premier agencement de verrouillage (36) vient en prise avec l'organe interne (28), et un deuxième état entièrement en prise dans lequel le deuxième agencement verrouillage (38) vient en prise avec l'organe interne (28) ;
et dans lequel
l'organe interne (28) peut tourner à la fois dans la première direction (A) et dans une deuxième direction (B) opposée à la première direction (A) lorsque le connecteur (12) est dans l'état désolidarisé, dans lequel l'organe interne (28) est empêché de tourner dans la première direction (A) lorsque le connecteur (12) est dans le premier état entièrement en prise ; et dans lequel l'organe interne (28) est empêché de se rétracter proximalement hors du boîtier (16) tout en permettant à l'organe interne (28) de tourner librement dans le deuxième état entièrement en prise.

11. Procédé selon la revendication 10,
dans lequel l'au moins un organe de préhension (92, 94, 96, 98, 100, 102) comprend deux brides incurvées (92) s'étendant à partir d'une surface proximale (40) de l'organe interne (28), chaque bride incurvée (92) comportant une partie plane (93a) et une partie inclinée (93b, 93c) s'étendant à partir de chaque extrémité de la partie plane (93a), et
dans lequel les brides incurvées (92) sont configurées pour être saisies par l'utilisateur pour empêcher la rotation de l'organe interne (28) dans la deuxième direction (B).

12. Procédé selon la revendication 10,
dans lequel l'au moins un organe de préhension (92, 94, 96, 98, 100, 102) comprend deux brides (98) positionnées sur le connecteur (12), chaque bride (98) comprenant une partie verticale qui s'étend verticalement le long d'une surface latérale du connecteur (12), et
dans lequel les brides (98) sont configurées pour être saisies par l'utilisateur pour empêcher la rotation de l'organe interne (28) dans la deuxième direction (B).
